# EUROPEAN PATENT APPLICATION

(11) **EP 1 576 952 A1**
(43) Date of publication of application: **21.09.2005**
(21) Application number: 04075884.9
(22) Date of filing: 18.03.2004
(51) Int. Cl.: A61K 9/16, A61K 38/27

(54) **Hydrogel microspheres with improved release profile**

(71) Applicant: OctoPlus Technologies B.V., 2333 CL Leiden (NL)
(72) Inventor: Verrijk, Rudolf, 2202 HS Noordwijk (NL)
(74) Representative: Winckels, Johannes Hubertus F.

(57) **Abstract**

The invention provides an emulsion-based method for the preparation of controlled release microspheres for the delivery of active compounds. The method comprises the preparation of an emulsion comprising an aqueous dispersed phase which comprises a polymer capable of forming a hydrogel, a bioactive protein, and water, and which is substantially free from insoluble aggregates of the bioactive protein. Subsequently, the polymer physically or chemically crosslinked to form a hydrogel. The invention further provides active protein-loaded hydrogel microspheres which are prepared by the process, and which are substantially free from insoluble aggregates of the active protein. The microspheres exhibit controlled release, with release profiles which are considerably improved over those of previously known hydrogel microspheres. The microspheres may be used to deliver therapeutic or diagnostic proteins by injection.

## Description

### FIELD OF THE INVENTION

The invention relates to aqueous-based methods for preparing hydrogel systems, and preferably hydrogel microspheres, and addresses problems frequently associated with known methods in this field.

In another aspect, it relates to controlled or sustained release microspheres which are useful for drug delivery or diagnostic purposes, and to pharmaceutical and diagnostic compositions containing such microspheres. The microspheres of the invention are particularly useful for the delivery of bioactive proteins, many of which are difficult to incorporate in conventional microspheres. In another aspect, the invention deals with microspheres prepared from hydrogel-forming polymers, such as water-soluble polymers which can be physically or chemically crosslinked to form insoluble particles. In further aspects, the invention is related to the incorporation of high payloads of proteins in microspheres, to achieving desirable release profiles with slow release rates, little or no burst effect, reproducible performance, and to stability issues with regard to incorporated proteins.

### BACKGROUND OF THE INVENTION

Many bioactive proteins are difficult to incorporate in delivery systems and particularly in the delivery systems of the type mentioned above, such as conventional polymeric microspheres. Some of the difficulties relate to the preservation of the purity and bioactivity of the proteins. In addition, it is noted that there is a need for systems allowing the incorporation of high payloads of proteins in microspheres, to achieve desirable release profiles with slow release rates, little or no burst effect, reproducible performance, and to stability issues with regard to incorporated proteins.

Takada *et al*. describe in their article in J. Controlled Release (88 (2003), 229-242) biodegradable microcapsules for the sustained release of recombinant human Growth Hormone (rhGH). These capsules are prepared by a solid-in-oil-in-water emulsion solvent evaporation technique using lyophilized protein particles. The key of the process described is that "solid-state proteins retain their activity in organic conditions". Particularly, Takada *et al*. disclose an injectable sustained release formulation of rhGH by incorporating lyophilized rhGH powder into biodegradable copoly(DL-lactic/glycolic)acid (PLGA) microcapsules. The specific bioactivity of rhGH extracted from the microcapsules formed is said to be as potent as that of intact rhGH in a cell proliferation assay. The particle size of the proteins is said to be very important for microencapsulation as regards high entrapment and small initial release. In order to obtain lyophilized rhGH powder with small bulkiness and low static electricity induced by handling, certain amounts of ammonium acetate were added as a volatile buffer component. One of the major disadvantages of this method is that it requires the use of organic solvents.

WO-A-01/55360 and the corresponding US-A-2001/0048947 disclose microparticles containing insoluble biologically active substances, which are made in solvent evaporation processes, wherein the microparticle-forming polymer is dissolved in a volatile organic solvent such as methylene chloride. The active agent is incorporated in this solution, either in soluble form or dispersed in fine particles. This mixture is suspended in an aqueous phase that contains a surface active agent, such as poly(vinyl alcohol). While stirring, most of the organic solvent is evaporated, leaving solid microspheres. As an alternative, a biologically active substance is dispersed or dissolved in a volatile organic solvent that also contains the microsphere forming polymer. The mixture is suspended in oil and stirred to form an emulsion. As the solvent diffuses into the oil phase, emulsion droplets harden into microspheres. Moreover, a method is described wherein a homogeneous mixture of a biologically active substance and a polymerizable macromer are atomized to form fine droplets. These droplets are irradiated to form microspheres. Finally, a water-in-oil emulsion is formed containing a polymerizable macromer and an active ingredient; which emulsion is irradiated to form hydrogel particles. Among the disadvantages of this method is the requirement for organic solvents, the possibly negative impact of irradiation on the physiological tolerability of the product and the possibly negative effect on protein integrity.

Another type of controlled release microspheres is based on chemical hydrogels which are prepared by crosslinking suitable prepolymers. For example, WO 98/00170 discloses microspheres which are made from derivatised dextrans. These hydrogel microspheres are compatible with therapeutic proteins, but also exhibit some excellent physiological tolerability. They can be prepared without the use of organic solvents according to a process described in e.g. WO 98/22093.

However, all these known microspheres and the conventional methods for their preparation are associated with significant problems when it comes to the incorporation of bioactive proteins. As mentioned above, some of the problems may relate to the release behaviour, such as burst-release effects or incomplete drug release. One of the underlying causes of many of these problems may be the relative sensitivity of bioactive proteins. In contrast to small molecular entities which are also used in therapy and diagnostics, proteins possess a dynamic three-dimensional structure which is largely determined by weak non-covalent forces which are dependent on the environment of the molecule. Proteins easily lose their tertiary (or, if applicable, quaternary structure) under unfavourable conditions, such as in the presence of organic solvents. Unfortunately, these non-covalent structural changes are often irreversible, and thus constitute a first step of protein denaturation, which is often followed by other steps, such as irreversible precipitation.

Before conceiving the present invention, it was observed that even relatively mild conditions such as those described in the all-aqueous method of WO 98/22093 often, or at least sometimes lead to protein denaturation. In particular, bioactive proteins with limited water solubility tend to be negatively affected by the conditions typically selected for the preparation of hydrogel microspheres.

Thus, there is a need for further improved methods for preparing controlled release microspheres which are especially suitable for the incorporation of sensitive bioactive proteins.

### OBJECTS OF THE INVENTION

It is an object of the invention to provide a method for preparing controlled release microspheres which overcomes or at least reduces or minimizes the limitations and disadvantages of presently known methods with regard to the incorporation of bioactive proteins. In particular, it is an object of the invention to provide a method which leads to microspheres in which the incorporated protein is pure, bioactive and releasable.

It is another object to provide a method for the incorporation of bioactive proteins with limited water solubility into hydrogel microspheres.

It is an object of the present invention to provide a controlled release system on the basis of a preparation process wherein water is the medium for all constituents used. The advantages of using a water based system are that no systemically or environmentally unsafe organic solvents need to be used, and no solvents or phases that may lead to protein configuration changes that have an effect on the efficiency of the biologically active protein to be released.

It is a further object to provide active protein-loaded microspheres with improved release behaviour, and particularly without significant burst-effect or incomplete release.

In yet another aspect, it is an object of the invention to provide therapeutic and diagnostic compositions comprising such improved microspheres, and uses thereof.

Further objects of the invention will become clear in the light of the following description.

### SUMMARY OF THE INVENTION

The invention provides an emulsion-based method for the preparation of controlled release microspheres for the delivery of active compounds. The method comprises the steps of forming an emulsion comprising an aqueous dispersed phase which comprises a polymer capable of forming a hydrogel, a bioactive protein, and water, and subsequently crosslinking the polymer physically or chemically to form a hydrogel. The aqueous dispersed phase is further characterised in that it is substantially free from insoluble aggregates of the bioactive protein.

The absence of insoluble protein aggregates in the dispersed phase can be achieved, for example, by preparing the emulsion from pre-equilibrated liquid phases, or by preparing the dispersed phase by a method comprising the dissolution of freeze dried active protein in an aqueous carrier. In another aspect, the absence of insoluble protein aggregates can be achieved by carrying out the method under conditions which are selected to avoid the precipitation of the active protein before the crosslinking of the polymer to form hydrogel microspheres.

The invention further provides active protein-loaded hydrogel microspheres which are prepared by an emulsion-based process, and which are substantially free from insoluble aggregates of the active protein. The microspheres exhibit controlled release, with release profiles which are considerably improved over those of previously known hydrogel microspheres. Typically, the microspheres of the invention release at least about 90 or 95 % of their incorporated drug load in an active form during the predetermined release period. The microspheres may be used to deliver therapeutic or diagnostic proteins by injection.

### DETAILED DESCRIPTION OF THE INVENTION

The invention provides a method of preparing controlled release microspheres comprising the steps of (a) forming an emulsion comprising an aqueous dispersed phase, said dispersed phase comprising a polymer capable of forming a hydrogel, a bioactive protein, and water, and subsequently (b) crosslinking the polymer physically or chemically to form a hydrogel. The aqueous dispersed phase is further characterised in that it is substantially free from insoluble aggregates of the bioactive protein.

Furthermore, the invention provides microspheres for the controlled release of active proteins. The microspheres comprise at least one bioactive protein in a non-aggregated, dissolvable and releasable state. This is in contrast to many conventional protein-loaded microspheres, which contain the protein in an at least partially aggregated and denatured state. It is assumed by the inventors that many of the problems associated with conventional protein-loaded microspheres, such as undesirable release rates, burst-effects, incomplete release, and protein instability, are directly or indirectly associated with the presence of insoluble protein aggregates.

As used herein, "microspheres" are broadly defined as micron- or submicron-scale particles which are typically composed of solid or semi-solid materials and capable of carrying and releasing a bioactive compound. Microspheres may be more or less spherical, depending on the method of preparation. They also include particles with a structure comprising an inner core and an outer coat, such as nano- and microcapsules. In this respect, the terms "microspheres", "microparticles", and "microcapsules" may be used interchangeably. Typically, the weight-average diameter of the microspheres of the invention ranges from approximately 100 nm to approximately 500 µm. More preferably, the average particle diameter is between about 1 µm and about 100 µm. In another embodiment, the average diameter of the microspheres is between about 10 µm and about 80 µm, which is a very useful size range to achieve, for instance, local tissue retention of the spheres after intramuscular, subcutaneous, or intratumoral injection in case the spheres are used as a depot formulation providing for the sustained release of an incorporated active material.

As used herein, "controlled release" means any type of release which is not immediate release. For example, controlled release can be designed as modified, extended, sustained, delayed, prolonged, or constant (i. e. zero-order) release. In theory, one of the most useful release profiles is constant release over a predetermined period of time. In practice, such zero-order release is rarely achieved. In the context of this invention, controlled release typically refers to sustained release.

Sustained release, in this context, means that the respective microspheres release the incorporated bioactive protein so slowly that the duration of action is significantly prolonged in comparison with the administration of a solution of the same protein. In other words, a short-acting protein with an effective biological half-life of a few minutes may be formulated as a sustained release microsphere composition perhaps even with a release over a few hours only, while other proteins which are normally administered every one or two days may be released from sustained-release compositions over a period of weeks to months. In some rare cases, it may be desirable, and it is achievable with the present invention to release a protein from microspheres over several years. Preferred sustained release compositions according to the invention have release times from about one week to about 1 month.

An emulsion is defined as a system having at least two liquid phases, one of which is dispersed in the other. The dispersed phase is also referred to as inner phase, discontinuous phase, or incoherent phase, whereas the outer phase may also be referred to as coherent or continuous phase. Emulsions may comprise more than two phases. For example, they may be comprised of three liquid phases (i.e. triple emulsions), or two liquid phases and a solid phase. Common to all emulsions is that their outer phase is in a liquid state. If a third phase is present, such as a liquid or solid phase, this is usually dispersed in the dispersed phase which is dispersed in the outer phase.

In the context of the present invention, a polymer capable of forming a hydrogel is defined as a hydrophilic polymer which can be chemically or physically crosslinked to form a water-swollen, three-dimensional polymeric network. Usually, a hydrogel is in the solid or semi-solid state. Polymers which can be chemically crosslinked may represent prepolymers, which are defined as polymers carrying polymerisable groups (such as acrylic groups) so that they can act as monomers. Physical crosslinking means that the crosslinking does not involve the formation of new chemical bonds. For example, physical crosslinking may be achieved by the formation of crystalline regions or by multivalent ions whose charge is complementary to the charge of the polymer.

As used herein, a bioactive protein is a relatively large molecule or compound predominantly composed of amino acids which are linked to each other through peptide bonds, which may be used to diagnose, prevent or treat a disease or condition of a mammal or to change a function of an organism. A protein may also be referred to as polypeptide. There is no clear limit for the number of amino acids which a peptide must have to be a polypeptide or protein. Usually, the molecular mass of a polypeptide or protein is at least about 1,000, and more typically at least about 4,000 or 4 kDa. Most of the currently used therapeutic proteins have a molecular mass of more than 6 kDa. A bioactive protein may also comprise other chemical structures than amino acids. Biotechnology-derived proteins may be modified in posttranslation in the cells that they are expressed in. Glycosylation and sialylation are well-known examples of posttranslational modifications. Also, proteins are often modified with reactive substituents, linkers, or polymeric groups to change their properties. Pegylation, i.e. the attachment of an oligomeric or polymeric polyethylene glycol chain to the protein is one of the most successful techniques to improve the physicochemical and pharmacological properties of bioactive proteins. Thus, chemically modified or substituted proteins are also encompassed within the definition of a bioactive protein used herein.

As used herein, insoluble aggregates of a bioactive protein are aggregates which are not re-soluble under physiological conditions. It is not meant to include the naturally occurring bioactive aggregates of proteins, such as dimers, trimers or other multimers with retained bioactivity. For example, the cytosolic "120 kD protein" naturally occurs, and is active, as monomer, tetramer, and even as octamer. Instead, insoluble aggregates refer to degradation products of proteins resulting from the chemical or physical interaction of individual macromolecules in which the tertiary structure of the participating macromolecules (and, if applicable, their quaternary structure) has been substantially damaged. Macroscopically, aggregates may appear as precipitates, but not necessarily, and by no means it should be concluded that all protein precipitates are insoluble aggregates. Only those protein aggregates - whether macroscopic or colloidal - which are not re-soluble under physiological conditions are understood as insoluble aggregates in the context of the invention. Another characteristic of such insoluble aggregates is that they have largely lost their bioactivity.

In one of the preferred embodiments, the invention is carried out with a prepolymer being selected as the polymer capable of forming a hydrogel. Suitable prepolymers comprise macromolecules having polymerisable functional groups, such as groups comprising double bonds which are capable of undergoing radical polymerisation. Examples for such groups are vinyl or acrylic groups, including alkylacrylic groups. In a preferred embodiment, the prepolymer capable of forming a hydrogel is an alkylacrylated polymer, for example a methacrylated, hydroxyethacrylated, or hydroxyethylethacrylated polymer.

The backbone of the polymer or prepolymer should be substantially hydrophilic. Furthermore, it should be biocompatible in view of the intended use of the microspheres. It may be derived from naturally occurring, synthetic, or semisynthetic polymers. Among the naturally occurring polymers suitable for being selected as backbones are proteins and polysaccharides. Examples for proteins include gelatin, collagen, albumin, casein, and soy protein. Examples for polysaccharides include starch, amylose, amylopectin, cellulose, dextran, hyaluronate, alginate, pectin, acacia, carrageenan, guar gum, polydextrose, chitin, chitosan, tragacanth, and xanthan gum.

In a preferred embodiment, the backbone of the polymer or prepolymer is derived from dextran, which is hydrophilic and exhibits excellent biocompatibility. Furthermore, it is easily accessible, and can be tailored with regard to its substituents and modifications which lead to desirable properties such as the rate of biodegradation through hydrolysis. In another embodiment, dextran hydroxyethylmethacrylate (dexHEMA) is selected as polymer capable of forming a hydrogel. In further embodiment, a prepolymer composed of dextran as backbone, oligomeric spacers or side chains comprising lactide and/or glycolide units and hydroxyethylmethacrylate groups terminating the side chains is selected. These and other suitable prepolymers are described e. g. in WO 98/00170 and US 5,410,016, which documents are incorporated herein by reference for the description of suitable prepolymers (or macromers).

If, however, it is intended to carry out the method of the invention using physical crosslinking in order to form the hydrogel microspheres, the most useful polymers to be selected are not typically prepolymers as defined above. In this case, crosslinking does not involve a chemical reaction such as polymerisation. Non-covalent crosslinking can be achieved, for example, by selecting a polymer carrying one or more charges per macromolecule and a crosslinker selected from complementarily charged multivalent ions. For example, alginic acid may be ionically crosslinked with calcium ions, or with gelatin type B.

In another embodiment, a polymer is selected which is capable of forming crystalline regions, or crystallites, which act as physical crosslinks to form a three-dimensional hydrogel network. For example, certain polyvinylalcohols are known to be capable of forming crystallites under appropriate conditions. In one of the preferred embodiments, a crystallisable dextran is selected as polymer. Especially dextrans with a weight average molecular weight in the region of about 2,000 kDa to about 18,000 kDa are suitable within this embodiment, in particular dextrans with an average molecular weight in the region of about 6,000 kDa. Such dextrans and dextran derivatives are described in WO 02/17884, which is incorporated herein by reference.

In a further embodiment, the polymer is selected from those hydrophilic polymers which are capable of forming stereocomplexes. Stereocomplexes are racemic crystalline domains or regions composed of optically active, chiral monomeric units with opposite chirality. Stereocomplex hydrogels can, for instance, be formed by mixing enantiomerically enriched polymers of opposite chirality. Alternatively, they can be formed from only one polymeric species having regions of opposite chirality. For example, stereocomplex hydrogels can be formed from certain dextrans which are grafted with enantiomerically enriched side chains composed of lactic and/or glycolic acid units. Examples of stereocomplex hydrogels and polymers useful for making such hydrogels are described in WO 00/48576 and in the co-pending patent application EP 03078852.5 . The teachings contained in these documents are incorporated herein by reference.

In carrying out the invention, the bioactive protein is preferably selected from the group of therapeutic proteins. Typically, these molecules are too large to be bioavailable after non-invasive administration, or they may be unstable. Biotechnology and modern drug discovery techniques present an increasing number of such new drug compounds to the pharmaceutical industry. Particularly preferred compounds according to the invention are insulin, epoetin-alfa, epoetin-beta, calcitonin, heparin, IFN(interferon)-alfa-2a, IFN-alfa-2b, PEG-IFN-alfa, IFN-alfacon-1, IFN-beta, IFN-beta-1a, IFN-beta-1a, IFN-beta-1b, IFN-gamma-1b, somatropin, follitropin, menotropin, leuprolide, goserelin, buserelin, triptorelin, filgrastim (G-CSF), lenograstim (G-CSF), sargrarmostim (GM-CSF), PEG-G-CSF, blood clotting factors such as factor VIII and factor IX, nadroparin, dalteparin, tinzaparin, certoparin, reviparin, tirofiban, octreotide, monoclonal antibodies such as abciximab, as well as derivatives or functional analogues of any of these proteins.

The method of the invention is particularly useful when applied to the group of all-aqueous emulsion-based processes for making hydrogel microspheres. Earlier methods for making microspheres involved emulsions which also comprised at least one organic phase. However, organic solvents may lead to structural changes in protein structure, esp. in the secondary and tertiary structure. Such changes may lead to a denaturation of the protein drug. Since these structural changes normally lead to a loss in pharmacological activity and the occurrence of undesired side-effects, such changes are undesirable, as will be apparent. Moreover, the use of organic solvents is not desirable from an environmental point of view, either. Furthermore, it is hardly possible to avoid that traces of organic solvents will remain in or on the microspheres produced. Especially, when toxic solvents are used, such as the widely applied solvents chloroform and dichloromethane, this is a significant disadvantage.

More recently, emulsion-based methods for making microspheres were described which completely avoided the use of organic solvents, such as in WO 98/22093 and WO 03/035244, which documents are incorporated herein by reference. In short, these methods typically comprise the formation of an aqueous two-phase system in which a polymer capable of forming a hydrogel and, optionally, an active compound, is present in the dispersed phase, whereas the outer phase comprises a compound which, in combination with the polymer capable of forming a hydrogel and water, causes phase separation. Within the thus formed w/w-emulsion, the polymer is crosslinked to form the three-dimensional molecular network of a hydrogel.

A preferred compound which is used to induce aqueous phase separation in the presence of certain polysaccharides such as dextran or dextran derivatives is polyethylene glycol. According to this embodiment, an acrylated dextran prepolymer - most preferably dextran hydroxyethylmethacrylate - is present in the inner phase of the emulsion, while the outer phase comprises polyethylene glycol. Both the inner and the outer phase comprise water and, optionally, further excipients. Of course, the same principle can also be applied to aqueous two-phase systems comprising other incompatible components than hydroxyethyl methacrylated dextran and polyethylene glycol.

According to the present invention, the dispersed phase of the emulsion also contains the bioactive protein which is to be incorporated into the hydrogel microspheres. Whereas the documents referenced above describe the presence of the active compound in the dispersed phase of the emulsion only as one of the alternative ways of incorporating the drug substance, it has been found by the inventors that, for many protein drugs, this may be one of the most useful methods in order to achieve controlled release.

However, it is essential to select conditions which ensure that, at this stage of the microsphere preparation process, i.e. when the emulsion is formed, the dispersed phase is kept substantially free from insoluble aggregates of the bioactive protein. This means in essence that conditions are selected which ensure that either no insoluble aggregates of the protein are introduced into the system, and that no insoluble aggregates are generated during the process.

It is assumed by the inventors that many of the problems associated with conventional protein-loaded microspheres, such as undesirable release rates, burst-effects, incomplete release, and protein instability, are directly or indirectly associated with the presence of protein aggregates.

In fact, aggregation may be considered as one of the most common physical or physicochemical inactivation mechanisms of proteins in vitro, such as during the preparation and storage of pharmaceutical protein formulations, and the prediction of occurrence and degree of protein aggregation may be even more difficult than that of chemical degradation, such as deamination and oxidation (J. L. Cleland *et al*. Crit. Rev. Ther. Drug Carrier Syst. 10, 307-377, 1993).

By "substantially free" is meant that insoluble aggregates may be present, but only to a limited degree: most of the active substance must be present in an non-aggregated, dissolved or dissolvable form. Preferably, at least about 90 wt.-% of the active protein present in the dispersed phase of the emulsion is dissolved or dissolvable under physiological conditions, and no more than about 10 wt.-% of the protein is aggregated and/or denatured. In another preferred embodiment, no more than about 5 wt.-% of the active protein present in the dispersed phase of the emulsion is in the form of insoluble aggregates. In further embodiments, the content of insoluble aggregates is no more than about 3, 2 or 1 wt.-% of the total protein content of the dispersed phase, respectively.

The state of the protein, i.e. whether it is present in form of insoluble aggregates or not, can be determined by various methods or method combinations selected from SDS-page, FT-IR, size exclusion chromatography, fluorimetry, turbidimetry, particle size analysis using light scattering, and dissolution testing.

It has been found by the inventors that without observing the teachings of the present invention, the phase separation process described above, e.g. induced by a dextran derivative and polyethylene glycol in the presence of an active protein, typically leads to the formation of insoluble aggregates and thus to a substantial degree of denaturation of the protein. According to a hypothesis presently favoured by the inventors to explain this effect, the formation of protein aggregates may be caused by, or related to, the rapid migration of water from the nascent dispersed phase containing the protein into the nascent outer phase containing the highly hydrophilic polyethylene glycol. Thus, the fast hydration of the polyethylene glycol may occur at the cost of the solvatation of the protein, which is rapidly concentrated, whereby its tertiary structure may be negatively affected. In particular, proteins which have a relatively low water solubility seem liable to losing their tertiary structure and forming insoluble aggregates under such conditions.

In contrast, the dehydration of the protein should be avoided according to the present invention. In one of the preferred embodiments, this is achieved by preparing the w/w-emulsion from two aqueous phases which have been pre-equilibrated with regard to their water content. In other words, the step of forming the emulsion can be conducted by carrying out the following substeps: (a) providing a first aqueous phase comprising the bioactive protein, the polymer capable of forming a hydrogel, and water; (b) providing a second aqueous phase comprising the compound which is capable of phase separation and water, wherein the water content is at least at equilibrium with the water content of the first aqueous phase, and (c) combining the first and the second phase under conditions selected to emulsify the first aqueous phase in the second aqueous phase.

In this context, pre-equilibration means that the water content of each of the first and the second aqueous phase are calculated and selected to be at equilibrium. In other words, when the two phases are brought into contact with each other, no net water migration from one phase to the other will occur. Alternatively, the water content of the second phase which comprises the compound capable of inducing phase separation, e.g. polyethylene glycol, is selected to be higher than the equilibrium with the first phase would require. In this case, a net water migration from this second phase to the first phase will occur when the two phases are mixed. In both cases, no desolvatation of the bioactive protein which is present in the first phase takes place.

In systems with complex compositions, it may be difficult to pre-calculate the equilibrium water concentration for both aqueous phases. In these cases, it may be easier to determine the equilibrium water concentration empirically. To do so, the aqueous two-phase system can be prepared without observing the teachings of the present invention. Subsequently, samples of each of the two phases of the emulsion are withdrawn and analysed for their water content. Also, the volume of each of the phases is determined. From these data, it is easy to calculate the composition of each of the two phases.

In another embodiment, the aqueous two-phase emulsion is formed by carrying out the steps of (a) providing an aqueous phase having a temperature T₁, said phase comprising an amount of the bioactive protein, the polymer capable of forming a hydrogel, the compound which is capable of phase separation, and of water, wherein the amounts are selected to yield a single phase system at T₁, but a two-phase system at T₂, and wherein T₂ is lower than T₁, and (b) cooling the aqueous phase provided in step (a) from T₁ to T₂, thereby inducing phase separation.

This embodiment makes use of the fact that phase separation within an aqueous system does not only depend on the relative concentration of the dissolved components which induce phase separation, such as dextran and polyethylene glycol; temperature and pressure are also factors which influence the state of the system. Consequently, the same aqueous composition may exist as a one-phase system at one temperature, but as a two-phase system at a second temperature. In virtually all known cases, the second temperature at which the two-phase system exists is lower than the first temperature at which the single-phase systems exists. This is also true for the preferred polymers according to the invention, i.e. dextrans and dextran derivatives, in combination with polyethylene glycol.

Accordingly, the w/w-emulsion described in claim 1 can e.g. be prepared by mixing all components at an elevated temperature selected to yield a single aqueous phase. The elevated temperature resembles T₂ and is preferably selected in the range of about 30 to 80 °C, and in another preferred embodiment in the range of about 35 to 60 °C. Subsequently, the mixture is cooled, preferably to room temperature or to a temperature between about 0 °C and room temperature, to produce the emulsion. In order to prevent the rapid desolvatation of the bioactive protein, the cooling step should be conducted very slowly and carefully. A slow phase separation may be sufficiently mild for many bioactive proteins to allow their tertiary structure to be retained, and to prevent the formation of insoluble protein aggregates.

In a further embodiment of the invention, the preparation of the emulsion is conducted in such way that the bioactive protein is provided as a soluble solid. In particular, the step of forming the emulsion includes the substeps of (a) providing the bioactive protein in a solid, soluble form, and (b) combining the bioactive protein with water. In contrast to insoluble protein aggregates, soluble solid forms of the protein preserve the tertiary structure and bioactivity of the macromolecule.

Examples of solid forms which can be prepared carefully and with retained bioactivity are lyophilised powders or lyophilised single units, and soluble precipitates. Optionally, the solids may also contain other excipients. In one of the embodiments, the bioactive protein is provided as a lyophilisate which also contains the polymer from which the hydrogel is formed by crosslinking, e.g. dextran or a dextran derivative. Such a lyophilisate can be dispersed in an aqueous solution of polyethylene glycol, and as the polymer and the active protein dissolve and become hydrated, they will form a second aqueous phase which is dispersed in the outer polyethylene glycol phase.

The water with which the solid bioactive protein is combined may represent a single phase, or it may constitute an aqueous emulsion. In fact, it may be useful for the incorporation of certain, sensitive proteins to first prepare an emulsion which comprises all components of the emulsion of the invention except for the bioactive protein, e.g. an emulsion having a continuous phase primarily composed of polyethylene glycol and water and a dispersed phase predominantly composed of a dextran derivative - such as dextran hydroxyethyl methacrylate - and water, and to combine the solid, soluble active protein with this emulsion. Upon mixing, the active protein, which will typically have a much higher affinity to the dextran than to the polyethylene glycol phase, will become dissolved or dispersed in the inner phase of the emulsion. Again, the formation of insoluble protein aggregates is largely avoided.

Alternatively, the solid bioactive protein may be dispersed or dissolved in an aqueous solution of the compound which, in combination with water and with the polymer capable of forming a hydrogel, induces phase separation, e.g. polyethylene glycol.

In one of the preferred embodiments, the amount of water selected for dispersing or dissolving the bioactive protein is selected to be relatively low. More specifically, it is selected to be equivalent or less than that required for arriving at a protein concentration which is the same as that in the aqueous dispersed phase of the emulsion, which may be precalculated or empirically determined. In other words, a relatively concentrated aqueous solution or dispersion of the bioactive protein is prepared for the purpose of introducing the protein into the emulsion in such a way that the protein will not be further concentrated during the formation of the emulsion.

As mentioned above, it is important that the bioactive protein is not dehydrated under harsh conditions. The risk that rapid dehydration will have a negative impact on the tertiary structure of the protein along with aggregation, denaturation, and loss of bioactivity, is believed to be highest for those proteins which possess a moderate or low water solubility. Such proteins are defined as poorly soluble within the context of the present invention. A protein is poorly soluble if its solubility in water or physiological buffer solution is low relative to its dose in which the protein is to be incorporated in the microspheres. In other words, the teachings of the invention according to this embodiment may be applied with particular advantage to moderately soluble proteins which are administered in high doses, but also to very poorly soluble proteins which are administered in low doses.

Often the useful therapeutic proteins in the invention are also poorly soluble in absolute terms, having a solubility in water or in a physiological buffer solution (such as PBS buffer pH 7.4) of less than about 10 wt.-% at room temperature. In another embodiment, the bioactive protein has a solubility of less than about 5 wt.-%, and the invention is particularly useful for the incorporation of proteins with a solubility of less than about 2 wt.-%, or even less than 1 wt.-%, in controlled release microspheres.

Examples of proteins which may be successfully delivered with the microspheres of the invention include abciximab, agalsidase alfa, agalsidase beta, aldesleukin, alemtuzumab, alteplase, amylase, anistreplase, antithrombin III, aprotinin, argipressin, and asparaginase. Particularly suitable proteins preferred in the present invention include human growth hormone, somatropin, epoetin-alfa, epoetin-beta, G-CSF, and GM-CSF.

For any of the proteins mentioned herein, native and recombinant versions may exist. Both types of proteins can be selected according to the invention. In one of the preferred embodiments, the bioactive protein is selected from the group of recombinant proteins, such as rhGH.

In a further embodiment, the invention comprises the presence of at least one additional active ingredient which may or may not be a bioactive protein in the emulsion of the invention. The additional active ingredient is also incorporated within the controlled release microspheres. Alternatively, it may be added at a later stage of the preparation process for the microspheres. In this way, it may e.g. become incorporated into an aqueous carrier phase which serves as a suspension medium for the controlled release microspheres. If the additional active compound is incorporated into the microspheres, it will also be released in a controlled fashion, even though the release profile may differ substantially from that of the (first) bioactive protein.

Observing the teachings of the invention, such as by preparing the emulsion of the invention by the incorporation of a solid, but re-soluble, bioactive protein in finely divided form, and the avoidance of unfavourable conditions causing irreversible aggregation and denaturation, may allow a higher drug load to be incorporated into the microspheres. In fact, it is thus possible to disperse up to about 30 wt.-% of bioactive protein in the inner phase of the emulsion which also comprises the polymer capable of forming a hydrogel. Depending on the particular properties of the selected protein, even more than 30 % may be incorporated without loss of bioactivity.

In a preferred embodiment, the dispersed phase comprises about 1 to 30 wt.-% bioactive protein. In another preferred embodiment, the dispersed phase comprises about 5 to 25 wt.-% active protein.

The content of the polymer capable of forming a hydrogel is selected to provide a suitable ratio of polymer to active ingredient; at the same time, the selection needs to take into account the physicochemical nature of the polymer and the compound which, in combination with the polymer, causes phase separation in an aqueous system. In the case of dextran hydroxyethylmethacrylate and polyethylene glycol, it is preferred that the dispersed phase of the emulsion comprises at least about 30 wt.-% of the prepolymer. In another preferred embodiment, the dispersed phase comprises about 5 to 60 wt.-% of prepolymer.

After the crosslinking of the polymer which leads to the formation of the three-dimensional polymeric hydrogel network, the dispersed droplets of the inner phase of the emulsion solidify. By subsequent washing and drying, microspheres can be collected in which the active protein is incorporated. In these microspheres, the relative amount of active protein is higher than in the aqueous dispersed phase of the emulsion because the water content of the microspheres is lower. Typically, the microspheres of the invention comprise about 2 to 60 wt.-% of bioactive protein. More preferably, the protein content is about 5 to 30 wt.-%, and in particular about 5 to 20 wt.-%.

Following the teachings of the invention leads to microspheres with improved properties, particularly with improved release profiles. First of all, designing the preparation process in such a way that the intermediate product, i.e. the emulsion used in the process of the invention, is substantially free of insoluble protein aggregates, is an excellent way to create microspheres which also contain no such aggregates. In other words, the invention provides microspheres which contain an active protein predominantly in its pure, non-aggregated, non-denatured form. In such form, the protein is dissolvable and releasable under physiologic conditions. By "predominantly" is meant that at least about 90 wt.-% of the incorporated active protein is dissolvable and releasable from the microspheres under physiologic conditions, i. e. in native or simulated body fluids at body temperature and normal pressure. In another preferred embodiment, at least about 95 wt.-% are dissolvable and releasable. More preferably, at least about 97 wt.-% of the incorporated protein is dissolvable and releasable, and in another preferred embodiment, at least about 98 or 99 wt.-% is dissolvable and releasable.

The non-aggregated state of the protein within the microspheres may be determined by methods such as differential scanning calorimetry (DSC), microcalorimetry, or FT-IR spectroscopy. The dissolvable state can be determined by dissolution tests. The method of choice will much depend on the individual protein and the polymer composition of the microspheres.

After the microspheres are formed, they are typically washed or purified. This may be carried out e.g. as a filtration step using tangential flow filtration. The filtration step serves the purpose of separating the microparticles in dispersion from any impurities or other substances that are undesired in the final product. Impurities, for example, may be any substances that entered the microparticle dispersion as impurities contained in raw materials, or soluble degradation products that were produced during the step of microparticle formation. Other substances that need to be removed include those materials that were needed in any previous process step, but which are undesired in the product. Examples for such substances are crosslinking reagents, initiators, reaction product, degradation products, stabilizers, surfactants, detergents, thickeners, solvents, cosolvents, substances for adjusting the pH, osmotic pressure, ionic strength, or zeta potential, etc.

Another desirable effect that may need to be achieved during the purification step is the sizing of the microparticles, which in this case means that particles that are smaller than the desired size are removed by filtration. Preferably, microspheres with mean weight-average diameters of about 1 µm and about 100 µm are collected. In another embodiment, the average diameter of the microspheres is between about 10 µm and about 80 µm, and even more preferably in the region of about 30 to 80 µm.

In many cases, the active compound contained in the microparticle dispersion will not be stable enough to be stored in the presence of water for a practical period of time, permitting a commercially attractive shelf life. In these cases it is preferred that the dispersion is dried or freeze-dried for storage soon after the last purification step. A detailed description of the processing options is e.g. found in WO 03/035244, whose disclosure is incorporated herein by reference.

In freeze-drying, which is also referred to as lyophilization, a liquid solution or dispersion is rapidly frozen. While the sample is still in the frozen state, the pressure is reduced to such an extent that the solvent sublimes, i.e. it passes directly from the solid to the vapour state and is thus removed from the sample. During sublimation, the temperature may be elevated again. Lyophilization yields highly porous solid foams or powders which are easily and rapidly dissolved or redispersed. The freeze-drying step can be conveniently carried out after filling portions of the microparticle dispersion into the final containers, such as vials. The filling and freeze-drying steps are preferably conducted under aseptic conditions. After freeze-drying, the final containers are sealed. As the product thus obtained is a pharmaceutical or diagnostic product, it may be useful to package it within a secondary package that also contains a vessel with the reconstitution liquid, so that the product is provided as a kit. In such a kit, the dry solid containing the microparticles which is obtained by freeze-drying and the reconstitution liquid are either accommodated in individually sealed compartments formed within the same primary container, such as in a two-chamber syringe, or they may be stored in sealed compartments which are part of separate vessels.

The microspheres may be used advantageously as drug carriers in pharmaceutical or diagnostic compositions. In one embodiment, the invention provides such compositions. Preferably, the composition comprising the microspheres is an injectable sustained-release formulation suitable for parenteral administration, such as by intravenous, subcutaneous, cutaneous, intramuscular, intraarterial, intraperitoneal, locoregional, intralesional, or intratumoral injection or infusion. The composition will typically be a sterile aqueous liquid; for stability reasons, it may also be a dry composition, such as a powder, a lyophilized powder, a coherent lyophilized matrix, a dispersible tablet, or granules, which may be re-suspended in a sterile aqueous carrier prior to its use.

Typical excipients to formulate such compositions are known to pharmaceutical scientists and other formulation specialists. These excipients include substances to adjust the pH, such as acids, bases, buffer salts; compounds to adjust the tonicity, such as salts, sugars and sugar alcohols, amino acids; stabilizers and cryoprotectants, such as sugar alcohols, amino acids, and small peptides; antioxidants, preservatives, surfactants including phospholipids and poloxamers, etc. As required for parenteral administration, such compositions must be sterile, which is achieved either by aseptical processing or by sterilization after manufacture. Depending on the actual route of administration and on the desired release profile, the particle size must also be controlled to prevent undesired embolic effects through large microspheres, or lack of local retention of small spheres when a local depot effect is desired.

Another feature of the invention is associated with the shapes of the release profiles of the protein. In many cases, it is desirable to provide smooth and relatively constant release profiles. Initial burst release phases, unpredictable dose dumping effects, but also insufficiently high release rates during the later phases of release are undesirable release characteristics often encountered in the literature relating to experimental microsphere formulations.

As mentioned above, smooth and even release curves are much more easily achievable when microspheres are designed to contain a protein in a substantially non-aggregated state, or when they are substantially free from insoluble aggregates. It is presently believed that this advantage is also related to the fact that non-aggregated proteins have a uniform hydrodynamic radius, and that the preferred types of hydrogels can be adapted with regard to their pore size to control the release of the protein, whereas the presence of aggregates, which have a broader size distribution, leads to much more variability through the rapid release of some protein fractions and to the very slow release of larger aggregates. Actually, some protein aggregates may not be releasable from the microspheres at all, unless the hydrogel matrix degrades to a substantial degree.

In one of the embodiments of the invention, the microspheres prepared by the claimed method release no more than 20 wt.-% of the incorporated protein during the first 24 h of release, as measured in *in vivo* or in a suitable *in vitro* model at 37 °C. In another embodiment, not more than about 10 wt.-% of the incorporated protein are released during the first 24 h. Furthermore, microspheres are provided which release their incorporated protein completely - or nearly completely - during the intended release period, so that no more than about 5 to 10 wt.-% of the protein are still present in the microspheres after the termination of the desired therapeutic or diagnostic effect of the microsphere composition in the body.

Typically, the microsphere compositions are administered by injection, in particular by s.c. or i.m. injection. Alternatively, they may be administered by i.a., i.v., locoregional, intraperitoneal or intraarticular injection, or by nasal, pulmonary, oromucosal, vaginal administration. In certain few cases in which the protein can be absorbed from the gastrointestinal mucosa, the compositions may be administered orally as well.

The invention is further illustrating by the following examples which are, however, not presented with the intention to limit the scope of the invention:

### Example 1 (comparative example): Preparation of hGH-loaded microspheres with high content of insoluble aggregates.

Microspheres were prepared by a water-in-water emulsion technique. In detail, an aqueous solution of 0.2 g of 20 % (w/w) dextran hydroxyethylmethacrylate (dexHEMA, DS=16, MW_{dex}~40,000), containing 6 mg of dissolved human growth hormone (hGH), was added to 4.8 g of a 20 % (w/w) PEG solution (MW_{PEG}~10,000). The components were vigorously mixed for 1 minute by vortexing. The resulting emulsion was allowed to stabilise for 10 minutes. Subsequently, 180 µl of a KPS solution (50 mg/ml) and 100 µl of a 20% (v/v) TEMED solution (pH neutralized with 4M HCl) were added to start the polymerisation of the HEMA groups. The mixture was incubated for 30 minutes at room temperature without stirring, yielding crosslinked, solidified hydrogel microspheres. The microspheres were washed three times with PBS.

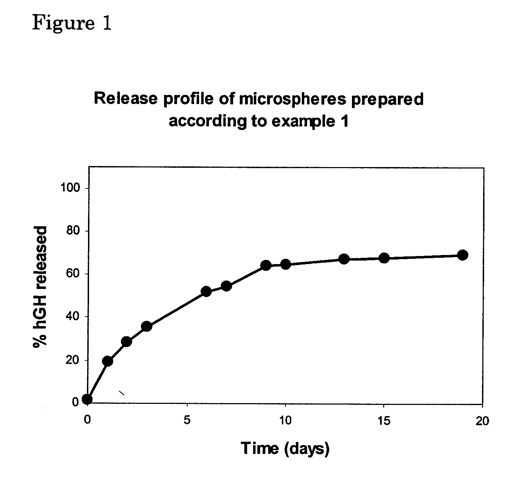

The spheres were tested in vitro for their drug release properties. The release experiments were conducted in PBS pH 7.4 at 37°C. Samples were analyzed for hGH by size exclusion chromatography, which measures dissolved hGH only. The cumulative release profile is shown in figure 1. After day 9 by which approximately 65 % release were achieved, there was very little further release indicating that a significant fraction of hGH was not releasable in soluble form.

### Example 2: Preparation of a w/w-emulsion using lyophilised hGH.

3 mg of solid, freeze dried hGH were mixed with 30 mg of a 50% (w/w) aqueous solution of dexHEMA (DS=16, MW_{dex}~40,000). After thoroughly mixing, 2.9 g of a 27 % (w/w) PEG solution (MW_{PEG}~10,000) was added. This was vigorously mixed for 1 minute by vortexing. The resulting emulsion was allowed to stabilise for 10 minutes. The absence of insoluble hGH aggregates was confirmed by size exclusion chromatography.

### Example 3: Preparation of a w/w-emulsion using a freeze dried mixture of hGH and dextran hydroxyethylmethacrylate (dexHEMA).

A solution of 2.5 mg of hGH and 30 mg of dex-HEMA (DS=16, MW_{dex}~40,000) was freeze dried in a vial. The freeze dried material was homogeneously mixed with 60 mg of water. Subsequently, 4.9 g of a 27 % (w/w) aqueous PEG solution (MW_{PEG}~10,000) were added. The blend was vigorously mixed for 1 minute by vortexing. The resulting emulsion was allowed to stabilise for 10 minutes. The absence of insoluble hGH aggregates was confirmed by size exclusion chromatography.

### Example 4: Preparation of a w/w-emulsion using a freeze dried mixture of hGH and dextran hydroxyethylmethacrylate (dexHEMA).

A solution of 2.5 mg of hGH and 30 mg of dexHEMA (DS=16, MW_{dex}~40,000) was freeze dried in one vial. Subsequently, 5 g of a 27 % (w/w) aqueous PEG solution (MW_{PEG}~10,000) were added. This blend was vigorously mixed for 1 minute by vortexing. The resulting emulsion was allowed to stabilise for 10 minutes. The absence of insoluble hGH aggregates was confirmed by size exclusion chromatography.

### Example 5: Preparation and characterisation of hGH-loaded microspheres.

In individual experiments, the emulsions prepared according to the examples 2, 3 and 4 were used to prepare hGH-loaded hydrogel microspheres. 180 µl of a KPS solution (50 mg/ml) and 100 µl of a 20% (v/v) TEMED solution (pH neutralized with 4M HCl) were added to start the polymerisation of the HEMA groups. The mixture was incubated for 30 minutes at room temperature without stirring, yielding crosslinked, solidified hydrogel microspheres. The microspheres were washed three times with PBS.

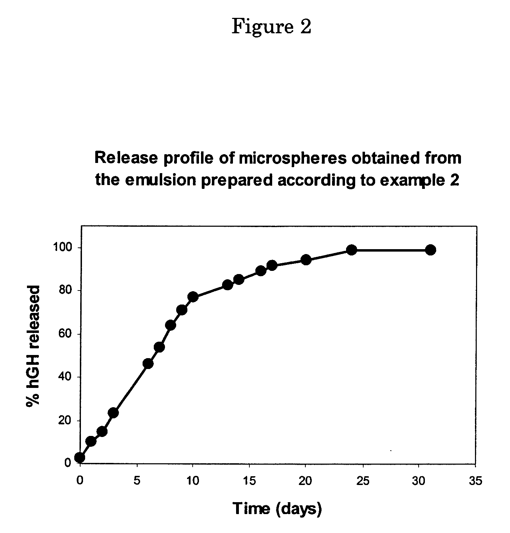

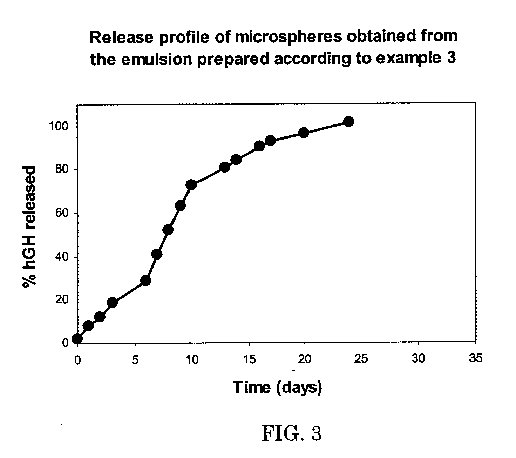

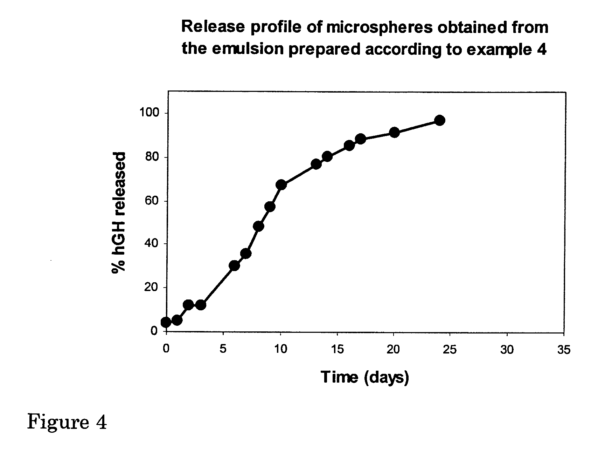

The spheres were tested in vitro for their drug release properties. The release experiments were conducted in PBS pH 7.4 at 37°C. Samples were analyzed for hGH by size exclusion chromatography, which measures dissolved hGH only. The cumulative release profiles of the microspheres prepared from the emulsions of examples 2, 3 and 4 are shown in figures 2, 3 and 4, respectively. The release profiles clearly indicate that hGH was incorporated in soluble and releasable form.

## Claims

1. A method of preparing controlled release microspheres comprising the steps of:
(a) forming an emulsion comprising an aqueous dispersed phase, said dispersed phase comprising:
- a polymer capable of forming a hydrogel,
- a bioactive protein, and
- water,
and subsequently
(b) crosslinking the polymer physically or chemically to form a hydrogel;
wherein the aqueous dispersed phase is substantially free from insoluble aggregates of said bioactive protein.

2. The method of claim 1, wherein the polymer capable of forming a hydrogel is a prepolymer.

3. The method of claim 1, wherein the polymer capable of forming a hydrogel is capable of being physically crosslinked by crystallisation or stereocomplex formation.

4. The method of any of the preceding claims, wherein the polymer capable of forming a hydrogel is a polysaccharide or modified polysaccharide, and preferably a modified dextran.

5. The method of any of the preceding claims, wherein the aqueous dispersed phase comprises about 5 to 60 wt.-% polymer or prepolymer, and about 1 to 30 wt.-% bioactive protein.

6. The method of any of the preceding claims, wherein the bioactive protein has a solubility of less than about 10 wt.-% in water or physiological buffer solution at room temperature.

7. The method of any of the preceding claims, wherein the bioactive protein is selected from the group consisting of insulin, epoetin-alfa, epoetin-beta, calcitonin, heparin, IFN(interferon)-alfa-2a, IFN-alfa-2b, PEG-IFN-alfa, IFN-alfacon-1, IFN-beta, IFN-beta-1a, IFN-beta-1a, IFN-beta-1b, IFN-gamma-lb, somatropin, follitropin, menotropin, leuprolide, goserelin, buserelin, triptorelin, filgrastim (G-CSF), lenograstim (G-CSF), sargrarmostim (GM-CSF), PEG-G-CSF, blood clotting factors such as factor VIII and factor IX, nadroparin, dalteparin, tinzaparin, certoparin, reviparin, tirofiban, octreotide, and monoclonal antibodies.

8. The method of any of the preceding claims, wherein the emulsion comprises an aqueous continuous phase.

9. The method of claim 8, wherein the aqueous continuous phase comprises a compound which, in the presence of water and of the polymer capable of forming a hydrogel, is capable of phase separation.

10. The method of claim 9, wherein the compound capable of phase separation is polyethylene glycol.

11. The method of any of the preceding claims, wherein the step of forming the emulsion comprises the substeps of:
(a) providing the bioactive protein in a solid, soluble form, and
(b) combining the bioactive protein with an amount of water.

12. The method of claim 11, wherein the amount of water is selected to yield a concentration of the bioactive protein which is about equivalent to, or higher than the concentration of the bioactive protein in the dispersed phase of the emulsion.

13. The method of claim 11, wherein the amount of water is provided in form of an aqueous solution or dispersion of a compound which, in the presence of water and of the polymer capable of forming a hydrogel, is capable of phase separation.

14. The method of any of claims 11 to 13, wherein the bioactive protein is provided in lyophilised form.

15. The method of claim 14, wherein the bioactive protein is provided as a lyophilised mixture comprising the polymer capable of forming a hydrogel.

16. The method of any of claims 11 to 13, wherein the bioactive protein is provided as a soluble precipitate.

17. The method of any of claims 11 to 16, wherein the step of forming the emulsion comprises the substeps of:
(a) providing a first aqueous phase comprising the bioactive protein, the polymer capable of forming a hydrogel, and water,
(b) providing a second aqueous phase comprising the compound which is capable of phase separation and water, wherein the water content is at least at equilibrium with the water content of the first aqueous phase,
(c) combining the first and the second phase under conditions selected to emulsify the first aqueous phase in the second aqueous phase.

18. The method of any of claims 11 to 17, wherein the step of forming the emulsion comprises the substeps of:
(a) providing an aqueous phase having a temperature T₁, said phase comprising an amount of the bioactive protein, the polymer capable of forming a hydrogel, the compound which is capable of phase separation, and of water, wherein the amounts are selected to yield a single phase system at T₁, but a two-phase system at T₂, and wherein T₂ is lower than T₁,
(b) cooling the aqueous phase provided in step (a) from T₁ to T₂, thereby inducing phase separation.

19. Controlled release microspheres comprising a biodegradable, physically or chemically crosslinked polymer and a bioactive protein, being substantially free from insoluble aggregates of said bioactive protein.

20. The microspheres of claim 19, wherein the crosslinked polymer is a polysaccharide, and preferably a dextran or dextran derivative.

21. The microspheres of claim 19 or 20, comprising about 2 to 60 wt.-% bioactive protein.

22. The microspheres of any of claims 19 to 21, wherein a fraction of at least about 95 wt.-% of the bioactive protein is dissolvable and releasable from the microspheres under physiological conditions.

23. The microspheres of any of claims 19 to 22, obtainable by the method of claim 1.

24. An injectable controlled release composition comprising the microspheres of any of claims 19 to 23.

25. The use of the microspheres of any of claims 19 to 23 as carriers for therapeutic or diagnostic bioactive proteins.
